# EUROPÄISCHE PATENTSCHRIFT

(11) **EP 1 644 314 B1**
(45) Veröffentlichungstag und Bekanntmachung des Hinweises auf die Patenterteilung: **10.08.2011**
(21) Anmeldenummer: 04740053.6
(22) Anmeldetag: 18.06.2004
(51) Int. Cl.: C07C 69/716, C07C 67/30

(54) **VERFAHREN ZUM HERSTELLEN VON DIFLUORACETESSIGS UREALKYLESTERN**
METHOD FOR PRODUCING DIFLUORO-ACETYL-ACETIC ACID ALKYLESTERS
PROCEDE POUR PRODUIRE DES ALKYLESTERS D'ACIDE DIFLUOROACETYLACETIQUE

(30) Priorität: 01.07.2003 DE 10329450; 11.07.2003 DE 10331496
(43) Veröffentlichungstag der Anmeldung: 12.04.2006
(73) Patentinhaber: Bayer Cropscience AG, 40789 Monheim (DE)
(72) Erfinder: GALLENKAMP, Bernd, 42113 Wuppertal (DE); MULDER, Lubbertus, 58135 Hagen (DE)
(86) Internationale Anmeldenummer: PCT/EP2004/006607
(87) Internationale Veröffentlichungsnummer: WO 2005/003077

(56) Entgegenhaltungen:
- DOLENCE J M ET AL: "Synthesis of Analogs of Farnesyl Diphosphate" TETRAHEDRON, ELSEVIER SCIENCE PUBLISHERS, AMSTERDAM, NL, Bd. 52, Nr. 1, 1996, Seiten 119-130, XP004104589 ISSN: 0040-4020
- PATENT ABSTRACTS OF JAPAN Bd. 0134, Nr. 27 (C-639), 22. September 1989 (1989-09-22) & JP 1 163143 A (KASHIMA SEKIYU KK), 27. Juni 1989 (1989-06-27)

## Beschreibung

Die vorliegende Erfindung betrifft ein neues Verfahren zum Herstellen von 4,4-Difluoracetessigsäurealkylestern (4,4-Difluor-3-oxobutansäurealkylestem) aus 4-Chlor-4,4-difluoracetessigsäurealkylestern, welche wiederum aus 2-Chlordifluoressigsäurealkylestem erhalten werden.

Es ist bereits bekannt, dass man 4,4-Difluoracetessigsäureethylester durch Umsetzung von Difluoressigsäureethylester mit Ethylacetat in Gegenwart von Natriumhydrid erhalten kann (vgl. Tetrahedron 2001, 57, 2689-2700). Die Ausbeute dieser Reaktion ist mit 25 % allerdings sehr unbefriedigend. Zudem ist der als Nebenprodukt erhaltene Acetessigsäureethylester nur schwer vom gewünschten Produkt abzutrennen.

Außerdem ist bekannt, dass sich 4,4-Difluoracetessigsäureethylester durch die Reaktion von Difluoressigsäureethylester mit Bromessigsäureethylester in Gegenwart von Zink herstellen lässt (vgl. Tetrahedron 1996, 52, 119-130). Auch bei diesem Verfahren lassen die Ausbeuten stark zu wünschen übrig.

Beiden genannten Verfahren ist darüber hinaus der Nachteil gemeinsam, dass der eingesetzte Difluoressigsäureethylester sehr teuer und somit für die großtechnische Produktion als Edukt unattraktiv ist.

Weiterhin ist bekannt, dass man eine Chlordifluoracetylgruppe als Substituent eines Aromaten unter Verwendung von Natrium-formaldehydsulfoxylat-Dihydrat reduzieren kann (vgl. Tetrahedron Lett. 2001, 42, 4811-4814). Jedoch lässt sich diese Reaktion nicht auf den 4-Chlor-4,4-difluoracetessigsäureethylester übertragen.

Die Aufgabe der vorliegenden Erfindung bestand also in der Bereitstellung eines neuen, wirtschaftlichen Verfahrens, durch welches 4,4-Difluoracetessigsäurealkylester mit hoher Gesamtausbeute und hoher Reinheit erhalten werden können.

Gegenstand der vorliegenden Erfindung ist also ein Verfahren zum Herstellen von 4,4-Difluoracetessigsäurealkylestem der Formel (I) 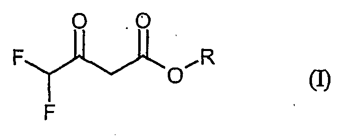 in welcher R für Alkyl steht,
dadurch gekennzeichnet, dass man
a) in einem ersten Schritt 4-Chlor-4,4-difluoracetessigsäurealkylester der Formel (II) 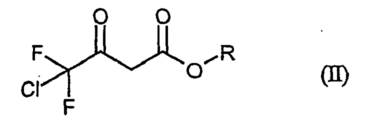 in welcher R die oben angegebene Bedeutung hat,
   mit Trialkylphosphiten der Formel (III)

   P(OR¹)₃ (III)

   in welcher
   - R¹: für C₁-C₄-Alkyl steht, wobei die Reste R¹ jeweils gleich oder verschieden sein können,
   umsetzt,
   die so erhaltenen Alkylphosphonsäureester der Formel (IV) 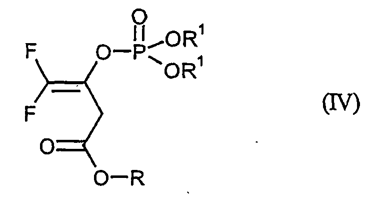 in welcher R und R¹ die oben angegebenen Bedeutungen haben,
   in einem zweiten Schritt mit einem Amin der Formel (V) 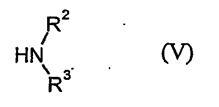 in welcher
   - R² und R³: unabhängig voneinander für Wasserstoff oder C₁-C₈-Alkyl oder gemeinsam für -CH₂-CH₂-O-CH₂-CH₂-, -CH₂-CH₂-S-CH₂-CH₂- oder -CH₂-CH₂-N(R⁴)-CH₂CH₂- stehen,
   - R⁴: für Wasserstoff oder C₁C₈-Alkyl steht,
   gegebenenfalls in Gegenwart eines Verdünnungsmittels umsetzt,
   und die so erhaltenen Enamine der Formel (VI) 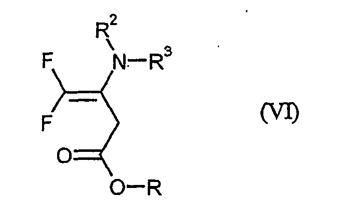 in welcher R, R² und R³ die oben angegebenen Bedeutungen haben,
   in einem dritten Schritt in Gegenwart einer Säure hydrolysiert.

Überraschenderweise lassen sich die Alkylphosphonsäureester der Formel (IV) nicht direkt durch saure Hydrolyse in das gewünschte Endprodukt überführen, vielmehr wird unter diesen Bedingungen Zersetzung beobachtet. Ebenfalls überraschend wird im zweiten Schritt des erfindungsgemäßen Verfahrens aus den Alkylphosphonsäureestern der Formel (IV) und einem Amin der Formel (V) nicht der gewünschte 4,4-Difluoracetessigsäurealkylester und das entsprechende Phophorsäureamid erhalten, sondern das Enamin der Formel (VI) und das Ammoniumsalz des Phosphorsäurediesters. Dieses Enamin wiederum lässt sich überraschend leicht durch saure Hydrolyse in die 4,4-Difluoracetessigsäureester der Formel (I) überführen. Dazu ist eine Isolierung des Enamins nicht einmal erforderlich.

Das erfindungsgemäße Verfahren überkommt also die oben genannten Nachteile bekannter Herstellungsverfahren und liefert 4,4-Difluoracetessigsäurealkylester in hoher Ausbeute und hoher Reinheit. Außerdem besitzt das Verfahren den Vorteil, dass sich Acetessigsäureester, welcher als Verunreinigung in 4-Chlor-4,4-difluoracetessigsäurealkylestern der Formel (II) enthalten sein kann, leicht aus dem Reaktionsgemisch entfernen lässt, Acetessigsäureester reagieren bei der Umsetzung mit Trialkylphosphiten der Formel (III) nicht und lassen sich von den Alkylphosphonsäureestern der Formel (IV) somit destillativ entfernen.

Ausgehend von 4-Chlor-4,4-difluoracetessigsäureethylester, Trimethylphosphit und Diisopropylamin kann das erfindungsgemäße Verfahren durch das folgende Formelschema veranschaulicht werden.

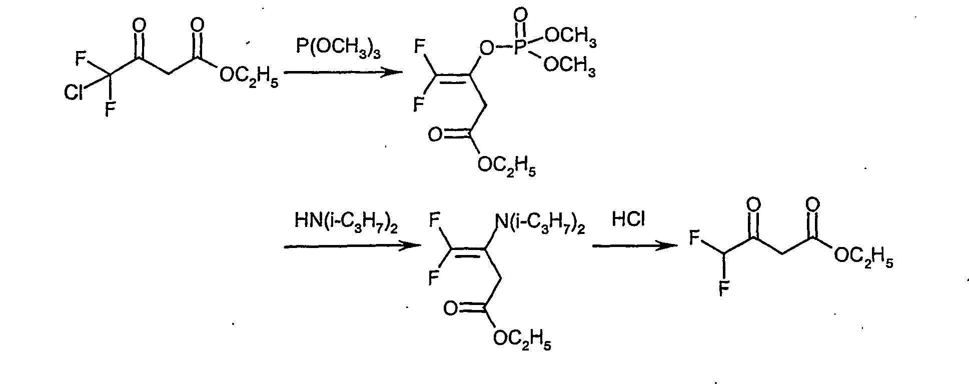

Die nach dem erfindungsgemäßen Verfahren erhältlichen 4,4-Difluoracetessigsäurealkylester können neben der in Formel (I) gezeigten Keto-Form auch in der Enol-Form vorliegen:

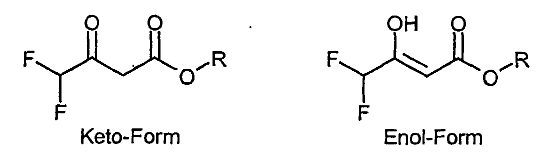

Neben den 4,4-Dißuoracetessigsäurealkylestern werden nach dem erfindungsgemäßen Verfahren auch die Hydrate der Verbindungen erhalten:

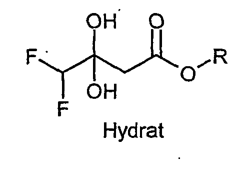

Als Produkt des erfindungsgemäßen Verfahrens wird daher neben 4,4-Difluoracetessigsäurealkyl-estern (in Keto- und Enol-Form) auch das jeweilige Hydrat verstanden. In Abhängigkeit von der Folgechemie können entweder alle drei Formen des Produktes oder jeweils nur bestimmte Formen weiter umgesetzt werden (vgl. unten).

Die im ersten Schritt des erfindungsgemäßen Verfahrens (a) als Ausgangsstoffe verwendeten 4-Chlor-4,4-difluoracetessigsäurealkylester der Formel (II) sind bekannt (vgl. Journal of Fluorine Chemistry 1992, 56, 271-284; Huaxue Xuebao 1983, 41, 729-729 und Chemical Abstracts 1984, 100, Abstract No. 22308; EP-A 0 082 252). Sie lassen sich beispielsweise herstellen, indem man
b) Chlordifluoressigsäurealkylester der Formel (VII) 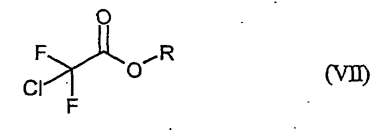 in welcher R die oben angegebene Bedeutung hat,
   mit Essigsäurealkylestern der Formel (VIII) 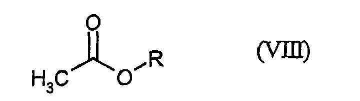 in welcher R die oben angegebene Bedeutung hat,
   in Gegenwart einer Base und in Gegenwart eines Verdünnungsmittels umsetzt.

Die Alkylphosphonsäureester der Formel (IV) und die Enamine der Formel (VI) sind neu. Sie.lassen sich gemäß dem erfindungsgemäßen Verfahren (a) herstellen.

Trialkylphosphite der Formel (III), Amine der Formel (V), Chlordifluoressigsäurealkylester der Formel (VII) (mögliche Herstellung siehe Herstellungsbeispiele) und der Essigsäurealkylester der Formel (VIII) sind bekannte Synthesechemikalien.

Im ersten Schritt des erfindungsgemäßen Verfahrens (a) werden 4-Chlor-4,4-difluoracetessigsäurealkylester der Formel (II) eingesetzt, in welcher R bevorzugt für C₁-C₈-Alkyl, besonders bevorzugt für C₁-C₆-Alkyl, ganz besonders bevorzugt für Methyl, Ethyl, n-, iso-Propyl, n-, iso-, sek-, tert-Butyl, und insbesondere ganz besonders bevorzugt für Methyl oder Ethyl steht.

Im ersten Schritt des erfindungs gemäßen Verfahrens (a) werden Trialkylphosphite der Formel (III) eingesetzt, in welcher R¹ jeweils gleich oder verschieden sein kann. R¹ steht bevorzugt für Methyl, Ethyl, n-, iso-Propyl, n-, iso-, sek-, tert-Butyl, besonders bevorzugt für Methyl oder Ethyl steht.

Bevorzugte Alkylphoshonsäureester sind solcher Verbindungen der Formel (IV), in welcher R die oben als bevorzugt, besonders bevorzugt, ganz besonders bevorzugt bzw. insbesondere ganz besonders bevorzugt angegebenen Bedeutungen hat, und in welcher R¹ jeweils gleich oder verschieden sein kann und die oben als bevorzugt bzw. besonders bevorzugt angegebenen Bedeutungen hat.

Im zweiten Schritt des erfindungsgemäßen Verfahrens (a) werden Amine der Formel (V) eingesetzt, in welcher R² und R³ unabhängig voneinander bevorzugt für Wasserstoff, C₁-C₆-Alkyl oder gemeinsam für -CH₂-CH₂-O-CH₂-CH₂-, -CH₂-CH₂-S-CH₂-CH₂- oder -CH₂-CH₂-N(R⁴)-CH₂-CH₂-, besonders bevorzugt für Wasserstoff, Methyl, Ethyl, n-, iso-Propyl, n-, iso-, sek-, tert-Butyl oder gemeinsam für -CH₂-CH₂-O-CH₂-CH₂-, ganz besonders bevorzugt unabhängig voneinander für iso-Propyl, iso-, sek-, tert-Butyl oder gemeinsam für -CH₂-CH₂-O-CH₂-CH₂, insbesondere ganz besonders bevorzugt jeweils für iso-Propyl stehen. In Formel (V) steht R⁴ bevorzugt für Wasserstoff oder C₁-C₆-Alkyl, besonders bevorzugt für Wasserstoff, Methyl, Ethyl, n-, iso-Propyl, n-, iso-, sek- oder tert-Butyl.

Bevorzugte Enamine sind solcher Verbindungen der Formel (VI), in welcher R die oben als bevorzugt, besonders bevorzugt, ganz besonders bevorzugt bzw. insbesondere ganz besonders bevorzugt angegebenen Bedeutungen hat, und in welcher R² und R³ die oben als bevorzugt, besonders bevorzugt bzw. ganz besonders bevorzugt angegebenen Bedeutungen haben.

### Erster Schritt des erfindungsgemäßen Verfahrens (a)

Der erste Schritt des erfindungsgemäßen Verfahrens (a) wird in der Regel ohne weitere Verdünnungsmittel durchgeführt. Es ist aber auch möglich, zusätzlich ein Verdünnungsmittel zu verwenden (z.B. Methylenchlorid).

Der erste Schritt des erfindungsgemäßen Verfahrens (a) kann innerhalb eines relativ großen Temperaturbereichs durchgeführt werden. Im Allgemeinen arbeitet man bei Temperaturen von 10°C bis 50°C, bevorzugt von 20°C bis 40°C, besonders bevorzugt von 25°C bis 30°C. Ganz besonders bevorzugt werden die Reaktionskomponenten im ersten Schritt bei 25°C bis 30°C zur Reaktion gebracht. Anschließend lässt man zunächst bei 40°C bis 45°C nachreagieren und auf Raumtemperatur abkühlen.

Die Reaktionszeit ist nicht kritisch und kann in Abhängigkeit von der Ansatzgröße in einem größeren Bereicht gewählt werden. Im Allgemeinen werden die Reaktanden über einen Zeitraum von bis zu 150 min, bevorzugt bis zu 120 min, besonders bevorzugt bis zu 90 min zusammen gebracht. Die Zeit für das Nachreagieren beträgt in der Regel 3 Stunden, während über Nacht (d.h. in ca. 16 Stunden) abgekühlt wird.

Die Aufarbeitung erfolgt nach üblichen Methoden. Im ersten Schritt wird in der Regel zunächst unter vermindertem Druck eingeengt und anschließend das Produkt dieses Schrittes durch Destillation gewonnen.

Bei der Durchführung des ersten Schrittes des erfindungsgemäßen Verfahrens (a) setzt man auf 1 Mol 4-Chlor-4,4-difluoracetessigsäurealkylester der Formel (II) im Allgemeinen zwischen 0,5 Mol und 5 Mol, vorzugsweise zwischen 0,5 Mol und 3 Mol, besonders bevorzugt zwischen 1 Mol und 2 Mol, ganz besonders bevorzugt zwischen 1,2 Mol und 1,7 Mol eines Trialkylphosphites der Formel (III) ein.

### Zweiter Schritt des erfindungsgemäßen Verfahrens (a)

Der zweite Schritt des erfindungsgemäßen Verfahrens (a) wird gegebenenfalls in der Gegenwart eines Verdünnungsmittels durchgeführt. Als Verdünnungsmittel kommen alle für solche Reaktionen üblichen inerten organischen Solventien infrage. Vorzugsweise verwendbar sind gegebenenfalls halogenierte, aliphatische, alicyclische oder aromatische Kohlenwasserstoffe, wie z.B. Petrolether, Hexan, Heptan, Cyclohexan, Methylcyclohexan, Benzol, Toluol, Xylol, Decalin, Chlorbenzol, Dichlorbenzol oder Dichlormethan; Ether, wie z.B. Diethylether, Diisopropylether, Methyl-tert butyl-ether, Methyl-tert-amylether, Dioxan, Tetrahydrofuran, 1,2-Dimethoxyethan, 1,2-Diethoxyethan oder Anisol; Nitrile, wie z.B. Acetonitril, Propionitril, n- oder iso-Butyronitril oder Benzonitril; Amide, wie z.B. N,N-Dimethylformamid, N,N-Dimethylacetamid, N-Methylformanilid, N-Methylpyrrolidon oder Hexamethylphosphorsäuretriamid; Sulfoxide, wie z.B. Dimethylsulfoxid; oder Sulfone, wie z.B. Sulfolan.

Der zweite Schritt des erfindungsgemäßen Verfahrens (a) kann innerhalb eines größeren Temperaturbereichs durchgeführt werden. Im Allgemeinen arbeitet man bei Temperaturen von 10°C bis 100°C, bevorzugt werden die Reaktionskomponenten bei Temperaturen von 20°C bis 30°C vermischt und anschließend bei 30 °C bis 100°C, bevorzugt bei 50°C bis 75°C zur Reaktion gebracht.

Die Reaktionszeit ist nicht kritisch und kann in Abhängigkeit von der Ansatzgröße in einem größeren Bereicht gewählt werden. Im Allgemeinen werden die Reaktanden über einen Zeitraum von wenigen Minuten bis zu 60 min, bevorzugt innerhalb von 10 min bis zu 30 min zusammen gebracht. Anschließend wird für mehrere Stunden, bevorzugt für bis zu 24 Stunden, besonders bevorzugt bis 20 Stunden reagieren gelassen.

Die Aufarbeitung erfolgt nach üblichen Methoden. Im zweiten Schritt wird in der Regel zunächst auf Raumtemperatur abgekühlt, mit Natriumchloridlösung und Wasser gewaschen, das Rohprodukt getrocknet und unter vermindertem Druck eingeengt. Das Enamin der Formel (VI) wird dann durch Destillation von weiteren Verunreinigungen befreit.

Bei der Durchführung des zweiten Schrittes des erfindungsgemäßen Verfahrens (a) setzt man auf 1 Mol Alkylphosphonsäureester der Formel (IV) im Allgemeinen zwischen 2,5 Mol und 5 Mol, vorzugsweise zwischen 3 Mol und 5 Mol, besonders bevorzugt zwischen 2 Mol und 4 Mol eines Amins der Formel (V) ein.

### Dritter Schritt des erfindungsgemäßen Verfahrens (a)

Die Hydrolyse im dritten Schritt des erfindungsgemäßen Verfahrens (a) wird in Anwesenheit einer Säure, bevorzugt von Schwefelsäure, Phosphorsäure oder Salzsäure, welche jeweils gegebenenfalls mit Wasser verdünnt werden, besonders bevorzugt von Salzsäure, ganz besonders bevorzugt von Mischungen aus Salzsäure und Wasser durchgeführt.

Der dritte Schritt des erfindungsgemäßen Verfahrens (a) kann innerhalb eines größeren Temperaturbereichs durchgeführt werden. Im Allgemeinen arbeitet man bei Temperaturen von 10°C bis 50°C, bevorzugt bei 20°C bis 30°C.

Die Reaktionszeit ist nicht kritisch und kann in Abhängigkeit von der Ansatzgröße in einem größeren Bereicht gewählt werden. Im Allgemeinen werden die Reaktanden über einen Zeitraum von wenigen Minuten bis zu 60 min, bevorzugt innerhalb von 10 min bis zu 30 min zusammen gebracht. Anschließend wird für mehrere Stunden, bevorzugt für bis zu 24 Stunden, besonders bevorzugt bis 20 Stunden reagieren gelassen.

Die Aufarbeitung erfolgt nach üblichen Methoden. Im dritten Schritt wird in der Regel zunächst mit einem geeigneten Lösungsmittel extrahiert, mit Natriumchloridlösung und Natriumhydrogencarbonatlösung gewaschen, das Rohprodukt getrocknet und unter vermindertem Druck eingeengt. Die 4,4-Difluoracetessigsäurealkylester der Formel (I) werden dann durch Destillation von weiteren Verunreinigungen befreit.

Bei der Durchführung des dritten Schrittes des erfindungsgemäßen Verfahrens (a) setzt man auf 1 Mol Enamin der Formel (VI) im Allgemeinen zwischen 0,5 Mol und 5 Mol, vorzugsweise zwischen 1 Mol und 5 Mol, besonders bevorzugt zwischen 1 Mol und 2,5 Mol an Säure ein.

### Erfindungsgemäßes Verfahren (b)

Im erfindungsgemäßen Verfahren (b) werden Chlordifluoressigsäurealkylester der Formel (VII) und Essigsäurealkylester der Formel (VIII) eingesetzt, in welchen R jeweils bevorzugt für C₁-C₈-Alkyl, besonders bevorzugt für C₁-C₆-Alkyl, ganz besonders bevorzugt für Methyl, Ethyl, n-, iso-Propyl, n-, iso-, sek-, tert-Butyl, und insbesondere ganz besonders bevorzugt für Methyl oder Ethyl steht.

Das erfindungsgemäße Verfahren (b) wird in Gegenwart einer geeigneten Base durchgeführt. Als solche kommen alle üblichen anorganischen oder organischen Basen infrage. Hierzu gehören vorzugsweise Erdalkalimetall- oder Alkalimetallhydride, -amide, -alkoholate, wie beispielsweise Natriumhydrid, Natriumamid, Lithium-diisopropylamid (LDA), Natrium-methylat, Natrium-ethylat, Kalium-tert.-butylat, besonders bevorzugt wird Lithium-diisopropylamid (LDA) und Natriumhydrid.

Das erfindungsgemäße Verfahren (b) wird in der Gegenwart eines Verdünnungsmittels durchgeführt.. Als Verdünnungsmittel kommen alle für solche Reaktionen üblichen inerten organischen Solventien infrage. Vorzugsweise verwendbar sind gegebenenfalls halogenierte, aliphatische, alicyclische oder aromatische Kohlenwasserstoffe, wie z.B. Petrolether, Hexan, Heptan, Cyclohexan, Methylcyclohexen, Benzol, Toluol, Xylol, Decalin, Chlorbenzol, Dichlorbenzol oder Dichlormethan; Ether, wie z.B. Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie z.B. Acetonitril, Propionitril, n- oder iso-Butyronitril oder Benzonitril; Amide, wie z.B. N,N-Dimethylformamid, N,N-Dimethylacetamid, N-Methylformanilid, N-Methylpyrrolidon oder Hexamethylphosphorsäuretriamid; Sulfoxide, wie z.B. Dimethylsulfoxid; oder Sulfone, wie z.B. Sulfolan.

Das erfindungsgemäße Verfahren (b) kann innerhalb eines größeren Temperaturbereichs durchgeführt werden. Im Allgemeinen arbeitet man bei Temperaturen von -80°C bis +100°C, bevorzugt bei -70°C bis 0°C.

Die Reaktionszeit ist nicht kritisch und kann in Abhängigkeit von der Ansatzgröße in einem größeren Bereicht gewählt werden. Im Allgemeinen werden die Reaktanden über einen Zeitraum von wenigen Minuten bis zu 180 min, bevorzugt innerhalb von 10 min bis zu 90 min zusammen gebracht. Anschließend wird für mehrere Stunden, bevorzugt für bis zu 24 Stunden, besonders bevorzugt bis 16 Stunden reagieren gelassen.

Die Aufarbeitung erfolgt nach üblichen Methoden. In der Regel zunächst neutralisiert, die Phasen getrennt, mit Natriumchloridlösung gewaschen, das Rohprodukt getrocknet und unter vermindertem Druck eingeengt. Die 4-Chlor-4,4-difluoracetessigsäurealkylester der Formel (II) werden dann durch Destillation von weiteren Verunreinigungen befreit.

Bei der Durchführung des erfindungsgemäßen Verfahrens (b) setzt man auf 1 Mol Chlordifluoressigsäurealkylester der Formel (VII) im Allgemeinen zwischen 0,5 Mol und 5 Mol, vorzugsweise zwischen 1 Mol und 5 Mol, besonders bevorzugt zwischen 1 Mol und 2,5 Mol an Essigsäurealkylester der Formel (VIII) ein.

Alle Schritte der erfindungsgemäßen Verfahren (a) und (b) werden im Allgemeinen unter Normaldruck durchgeführt. Es ist jedoch auch möglich, einzelne oder alle Schritte des erfindungsgemäßen Verfahrens unter erhöhtem oder vermindertem Druck - im Allgemeinen zwischen 0,1 bar und 50 bar, bevorzugt zwischen 1 bar und 10 bar - durchzuführen.

Die nach dem erfindungsgemäßen Verfahren (a) erhältlichen 4,4-Difluoracetessigsäurealkylester sind wertvolle Zwischenprodukte für die Herstellung von Difluormethyl-substituierte Pyrazolylcarbonsäure- bzw. Thiazolylcarbonsäure-Derivaten, welche wiederum Vorstufen von fungiziden Wirkstof fen darstellen (vgl. z.B. WO 02/08197 und DE-A 102 15 292).

Beispielsweise können 4,4-Difluoracetessigsäurealkylester zunächst mit Essigsäureanhydrid und Ortho-ameisensäuretrialkylester mit sehr guten Ausbeuten (bei Verwendung des Ethylester von über 90 %, vgl. Herstellungsbeispiele) zum Alkyl-2-(difluoracetyl)-3-alkoxyacrylat umgesetzt werden. Die Cyclisierung mit Methylhydrazin liefert 1-Methyl-3-difluormethyl-pyrazol-4-carbonsäure (im Falle des Ethylester in einer Ausbeute von über 65 %). Das falsche Isomer (1-Methyl-5-difluormethyl-pyrazol-4-carbonsäure) kann durch Kristallisation abgetrennt werden. Diese Umsetzung kann durch das folgende Formelschema veranschaulicht werden:

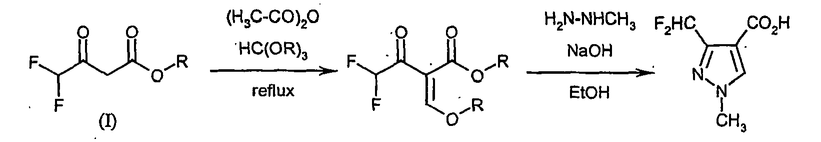

Außerdem können die 4,4-Difluoracetessigsäurealkylester zunächst chloriert werden, wodurch man das mono- und das dichlorierte Produkt (Alkyl-2,2-dichlor-4,4-difluor-3-oxobutanoat und Alkyl-2-chlor-4,4-difluor-3-oxobutanoat) erhält, welche beide mit Thioacetamid fast quantitativ zum 3-Methyl-4-difluormethyl-thiazol-5-carbonsäurealkylester umgesetzt werden können (vgl. folgendes Schema):

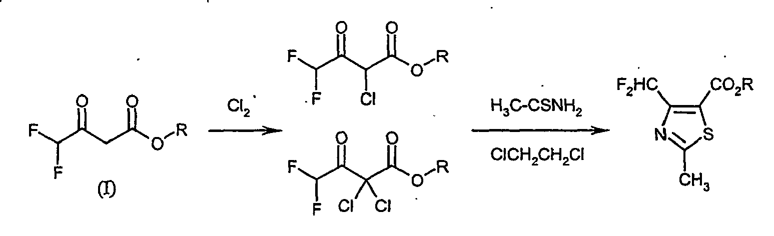

Das erfindungsgemäße Herstellen von 4,4-Difluoracetessigsäurealkylestern, sowie dessen Verwendung zum Herstellen von Difluormethyl-substituierten Heterocyclen wird in den nachstehenden Beispielen beschrieben, welche die obige Beschreibung weiter illustrieren. Die Beispiele sind jedoch nicht in einschränkender Weise zu interpretieren.

### Herstellunesbeispiele

### Bespiel 1:

### Schritt 1:

### Herstellung von Ethyl 3-[(dimethoxyphosphoryl)oxy]-4,4-difluorbut-3-enoat (IV-1)

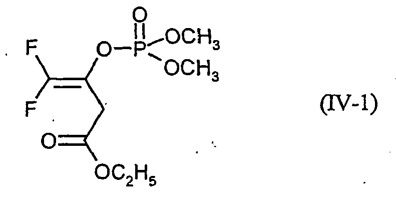

Zu 4-Chlor-4,4-difluoracetessigsäureethylester (305.1g, Gehalt 77.0%, 1.17mol) tropft man bei 25°C bis 30°C unter Eiswasserkühlung und Gasentwicklung innerhalb von 90 min Trimethylphosphit (232.0 g, 1.87 mol). Anschließend rührt man zuerst 1 h bei 30°C, dann 3 h bei 40°C bis 45°C nach. Zur Aufarbeitung wird auf Raumtemperatur abgekühlt (ca. 16 h) und unter vermindertem Druck eingeengt. Das Rohprodukt wird durch Destillation weiter gereinigt.

Man erhält 302.0 g (97%ig, 91 % der Theorie) an Ethyl 3-[(dimethoxyphosphoryl)oxy]-4,4-difluor-but-3-enoat (Siedepunkt 92-95°C bei 0.4 hPa).

### Schritt 2:

### Herstellung von Ethyl 3-(diisopropylamino)-4,4-difluorbut-3-enoat (VI-1)

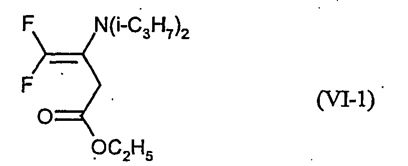

Zu einer Lösung von Ethyl 3-[(dimethoxyphosphoryl)oxy]-4,4-difluorbut-3-enoat (IV-1) (14.2 g, 97%ig, 0.05 mol) in 100 ml Methyl-tert-butyl-ether tropft man innerhalb von 10 min Diisopropylamin (15.2 g, 0.15 mol). Nach 19 h Kochen unter Rückfluss kühlt man auf Raumtemperatur ab, wäscht zweimal mit je 10 ml 10%iger Natriumchloridlösung, trocknet über Natriumsulfat, filtriert und engt unter vermindertem Druck ein. Der Rückstand wird zur weiteren Aufarbeitung destilliert.

Man erhält 8.8 g (95%ig, 67.4% der Theorie) an Ethyl 3-(diisopropylamino)-4,4-difluorbut-3-enoat (Siedepunkt 55-57°C bei 0.5 hPa).

### Schritt 2 und 3:

### Herstellung von 4,4-Difluoracetessigsäureethylester (I) ohne Isolierung des Ethyl 3-(diisopropylamino)-4,4-difluorbut-3-enoats (VI-1)

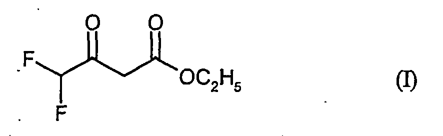

Zu einer Lösung von Ethyl 3-[(dimethoxyphosphoryl)oxy]-4,4-difluorbut-3-enoat (IV-1) (2570 g, 98.8%ig, 9.26 mol) in Methyl-tert butyl-ether (18.5 1) tropft man bei 20°C innerhalb von 10min Diisopropylamin (2811.6 g, 27.8 mol). Man rührt 20 h unter Rückfluß (57°C). Anschließend tropft man bei 20°C bis 25°C unter Kühlung eine Lösung von 2037 g konzentrierter Salzsäure in 4080 ml Wasser und rührt für 20 h nach. Es bilden sich zwei Phasen, die man trennt. Die wässrige Phase wird zweimal mit je 2.31 Methyl-tert-butyl-ether extrahiert. Die vereinigten organischen Phasen werden jeweils 2.8110%iger Natriumchloridlösung, 10%iger Natriumhydrogencarbonatlösung und nochmals 10%iger Natriumchloridlösung gewaschen, über Natriumsulfat getrocknet, filtriert und unter vermindertem Druck eingeengt. Das Rohprodukt wird durch Destillation aufgereinigt.
Man erhält 1179 g (92%ig, 76.6 % der Theorie) an 4,4-Difluoracetessigsäureethylester.

### Beispiel 2:

### Herstellung von 4-Chlor-4,4-difluoracetessigsäureethylester (II)

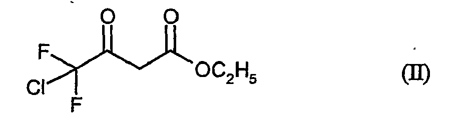

312.6 g (3.09 mol) Diisopropylamin werden in 1.55 1 Tetrahydrofuran gelöst und auf -70°C gekühlt. Zu dieser Lösung tropft man bei -60°C innerhalb von 80 min 852.9 g (3.08 mol) n-Butyllithium (2.5 molar in n-Hexan) und rührt für 45 min bei-70°C nach. Man lässt kurz auf-20°C kommen und kühlt sofort wieder auf -70°C ab. Bei -60°C tropft man innerhalb von 50 Min anschließend 264.3 g (3.0 mol) Essigsäureethylester. Dann tropft man innerhalb von 30 min bei derselben Temperatur 242.7 g Chlordifluoressigsäureethylester zu, rührt für 3 h bei -65°C bis -70°C nach und lässt dann auf Raumtemperatur kommen. Ab Erreichen von -5°C gibt man 1500 ml 4N HCl zu und lässt dann für 16 h stehen. Die wässrige Phase (pH 6-7) wird abgetrennt, die organische Phase mit 750 ml 2 N HCl und 1200 ml gesättigter Natriumchloridlösung gewaschen. Die organischen Phasen werden über Natriumsulfat getrocknet, filtriert und unter vermindertem Druck eingeengt. Zur weiteren Aufreinigung wird das Rohprodukt destilliert.
Man erhält 282.9 g (92%ig, 86.3 % der Theorie) an 4-Chlor-4,4-difluoracetessigsäureethylester.

### Beispiel 3:

### Herstellung von Chlordifluoressigsäureethylester (VII)

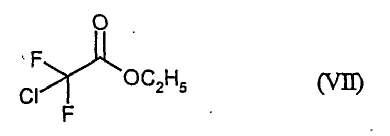

504.3 g (3.87 mol) Chlordifluoressigsäure und 5.0 g p-Toluolsulfonsäure werden in 775 ml Methylenchlorid gelöst und bei Raumtemperatur innerhalb von 30 min mit 311.6g (6.76 mol) Ethanol versetzt (Temperaturanstieg auf 33°C). Man rührt 38 h unter Rückfluss am Wasserabscheider nach und kühlt auf Raumtemperatur ab. Zur Aufarbeitung wäscht man mit Wasser (200 ml), gesättigter Natriumhydrogencarbonatlösung (200 ml) und erneut mit Wasser (200 ml), trocknet über Natriumsulfat, filtriert und destilliert das Lösungsmittel ab. Anschließend wird durch fraktionierte Destillation weiter aufgereinigt.
Man erhält 488.9 g (98%ig, 78.5 % der Theorie) an Chlordifluoressigsäureethylester (Siedepunkt 94-96°C).

### Beispiel 4:

### Herstellung von 1-Methyl-5-difluormethyl-pyrazol-4-carbonsäure

Zu einer Lösung aus 2394 g (10.35 mol) Ethyl-2-(difluoracetyl)-3=ethoxyacrylat in 5.4 1 Ethanol tropft man bei -15°C bis -5°C innerhalb von 3.5 h eine Lösung von 527.8 g (11.45 mol) Methylhydrazin in 0.71 Ethanol und rührt für 16 Stunden nach. Anschließend gibt man 560 g(14 mol) Natriumhydroxid und 3.5 1 Wasser zu und rührt 7 h bei 50°C. Das Reaktionsgemisch wird abgekühlt und unter vermindertem Druck eingeengt. Der Rückstand wird in 6 Wasser und 7 kg Eis aufgenommen und mit Dichlormethan gewaschen (einmal 3 1, einmal 2 1). Die eiskalte Wasserphase wird mit konzentrierter Salzsäure auf pH 2 eingestellt, das ausgefallene Produkt abgesaugt und im Vakuumschrank getrocknet. Das Rohprodukt wird in 8 1 Isopropanol (heiß) unter Rückfluss gelöst, anschheßend abgekühlt, 30 min bei 0°C bis 5°C gerührt, abgesaugt, mit 1.4 1 Isopropanol (5°C) nachgewaschen und bei 40°C im Vakuumschrank getrocknet.
Man erhält 1226.4 g (99.8%ig, 67.1 % der Theorie) an 1-Methyl-5-difluormethyl-pyrazol-4-carbonsäure [Log P (pH 2.3) = 0.52].

### Beispiel 5:

### Herstellung von 3-Methyl-4-difluormethyl-thiazol-5-carbonsäureethylester

Zu einem Gemisch aus Ethyl-2-chlor-4,4-difluor-3-oxobutanoat (50.4 %) und Ethyl-2,2-dichlor-4,4-difluor-3-oxobutanoat (68.2 g, 0.2 mol, 50.4 % Monochlorverbindung, 19.2 % Dichlorverbindung) in 500 ml 1,2-Dichlorethan gibt man 28 g (0.27 mol) Thioacetamid, kocht anschließend für 2 h unter Rückfluss und lässt dann 16 h stehen. Anschließend versetzt man langsam unter Rühren mit 300 ml gesättigter Natriumhydrogencarbonatlösung und trennt die Phasen. Die organische Lösung wird mit Natriumsulfat getrocknet und unter vermindertem Druck eingeengt. Die verbleibende Lösung wird filtriert, mit 20 ml Methylenchlorid nachgewaschen und unter vermindertem Druck eingeengt.
Man erhält 53.4 g (72%ig, 86.7 % der Theorie) an 3-Methyl-4-difluormethyl-thiazol-5-carbon-säureethylester [Log P (pH 2.3) = 2.18].

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

## Patentansprüche

1. Verfahren zum Herstellen von 4,4-Difluoracetessigsäurealkylestern der Formel (I) 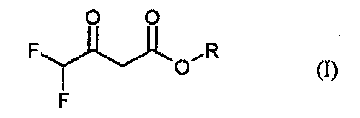 in welcher R für Alkyl steht,
**dadurch gekennzeichnet, dass** man
in einem ersten Schritt 4-Chlor-4,4-difluoracetessigsäurealkylester der Formel (II) 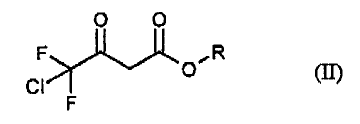 in welcher R die oben angegebene Bedeutung hat,
mit Trialkylphosphiten der Formel (III)
P(OR¹)₃ (III)
in welcher
R¹ für C₁-C₄-Alkyl steht, wobei die Reste R¹ jeweils gleich oder verschieden sein können,
umsetzt,
die so erhaltenen Alkylphosphonsäureester der Formel (IV) 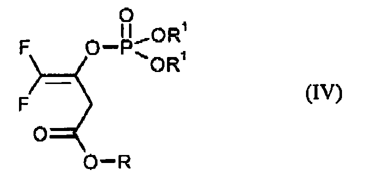 in welcher R und R¹ die oben angegebenen Bedeutungen haben,
in einem zweiten Schritt mit einem Amin der Formel (V) 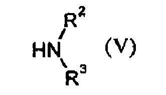 in welcher
R² und R³ unabhängig voneinander für Wasserstoff oder C₁-C₈-Alkyl oder gemeinsam für -CH₂- CH₂-O-CH₂-CH₂-, -CH₂-CH₂-S-CH₂-CH₂- oder -CH₂-CH₂-N(R⁴)-CH₂-CH₂- stehen,
gegebenenfalls in Gegenwart eines Verdünnungsmittels umsetzt, und die so erhaltenen Enamine der Formel (VI) 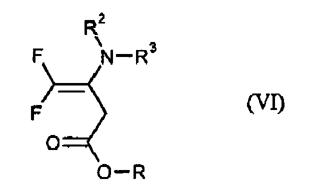 in welcher R, R² und R³ die oben angegebenen Bedeutungen haben,
in einem dritten Schritt in Gegenwart einer Säure hydrolysiert.

2. Verfahren gemäß Anspruch 1, **dadurch gekennzeichnet, dass** die im ersten Schritt als Ausgangsstoffe verwendeten 4-Ghlor-4,4-difluoracetessigsäurealkylester der Formel (11) hergestellt werden, indem man
chlordifluoressigsäunalkylester der Formel (VII) 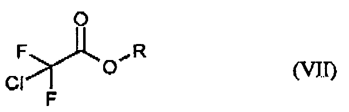 in welcher R die oben angegebene Bedeutung hat,
mit Essigsäursalkylestern der Formel (VIII) 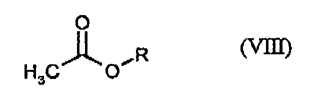 in welcher R die oben angegebenen Bedeutung hat,
in Gegenwart einer Base und in Gegenwart eines Verdünnungsmittels umsetzt.

3. Verfahren gemäß Anspruch 1 oder 2, **dadurch gekennzeichnet, dass** man Verbindungen der Formel (II) gemäß Anspruch 1 einsetzt, in welcher R für C₁-C₈-Alkyl steht.

4. Verfahren gemäß Anspruch 1 oder 2, **dadurch gekennzeichnet, dass** man Verbindungen der Formel (II) gemäß Anspruch 1 einsetzt, in welcher R für C₁-C₆-Alkyl steht

5. Verfahren gemäß Anspruch 1 oder 2, **dadurch gekennzeichnet, dass** man
Verbindungen der Formel (II) gemäß Anspruch 1 einsetzt, in welcher R für Methyl, Ethyl, n-, iso-Propyl, n-, iso-, sek-, tert-Butyl steht und
Verbindungen der Formel (III) gemäß Anspruch 1 einsetzt, in welcher R¹ für Methyl, Ethyl, n-, iso-Propyl, n-, iso-, sek-, tert-Butyl steht und
Verbindungen der Formel (V) gemäß Anspruch 1 einsetzt, in welcher R² und R³ unabhängig voneinander Wasserstoff Methyl, Ethyl, n-, iso-Propyl, n-, iso-, sek-, tert-Butyl oder gemeinsam für -CH₂-CH₂-O-CH₂-CH₂- stehen.

6. Verfahren gemäß Anspruch 1, 2, 3, 4 oder 5, **dadurch gekennzeichnet, dass** der erste Schritt ohne Verdünnungsmittel durchgeführt wird.

7. Verfahren gemäß Anspruch 1, 2, 3, 4, 5 oder 6, **dadurch gekennzeichnet, dass** die Hydrolyse im dritten Schritt in Anwesenheit von Schwefelsäure, Phosphorsäure oder Salzsäure, welche jeweils gegebenenfalls mit Wasser verdünnt werden, durchgeführt wird.

8. Alkylphosphonsäureester der Formel (IV) 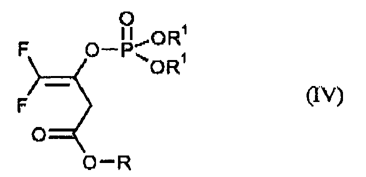 in welcher
R für Alkyl steht,
R¹ für C₁-C₄-Alkyl steht, wobei die Reste R¹ jeweils gleich oder verschieden sein können.

9. Enamine der Formel (VI) 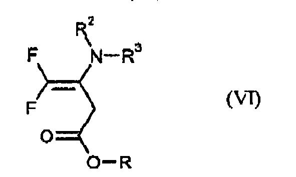 in welcher
R für Alkyl steht,
R² und R³ unabhängig voneinander für Wasserstoff oder C₁-C₈-Alkyl oder gemeinsam für -CH₂-CH₂-O-CH₂-CH₂-, -CH₂-CH₂-S-CH₂-CH₂- oder -CH₂-CH₂-N(R⁴)-CH₂-CH₂- stehen,

## Claims

1. Process for preparing 4,4-difluoroacetoacetic alkyl esters of the formula (I) 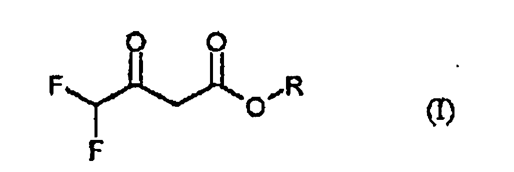 in which R is alkyl,
**characterized in that**
in a first step 4-chloro-4,4-difluoroacetoacetic alkyl esters of the formula (II) 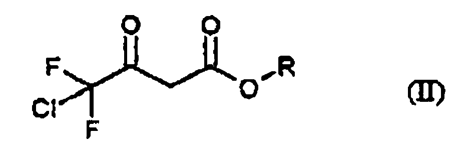 in which R is as defined above
are reacted with trialkyl phosphites of the formula (III)
P(OR¹)₃ (III)
in which
R¹ is C₁-C₄-alkyl, it being possible for the radicals R¹ each to be identical or different,
the resultant alkyl phosphonic esters of the formula (IV) 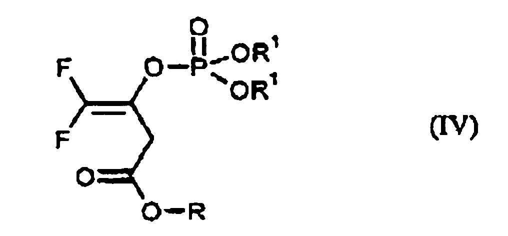 in which R and R¹ are as defined above are reacted in a second step with an amine of the formula (V) 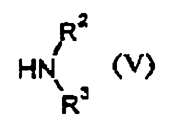 in which
R² and R³ independently of one another are hydrogen or C₁-C₈-alkyl or together are -CH₂-CH₂-O-CH₂-CH₂-, -CH₂-CH₂-S-CH₂-CH₂- or -CH₂-CH₂-N (R⁴) -CH₂-CH₂-,
optionally in the presence of a diluent,
and the resulting enamines of the formula (VI) 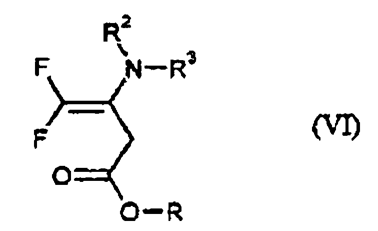 in which R, R² and R³ are as defined above are hydrolysed in a third step in the presence of an acid.

2. Process according to Claim 1, **characterized in that** the 4-chloro-4,4-difluroacetoacetic alkyl esters of the formula (II) that are used as starting materials in the first step are prepared by reacting
chlorodifluoroacetic alkyl esters of the formula (VII) 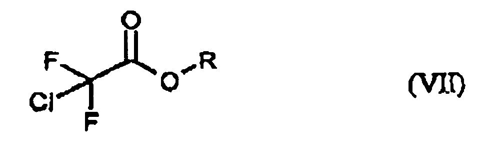 in which R is as defined above
with acetic alkyl esters of the formula (VIII) 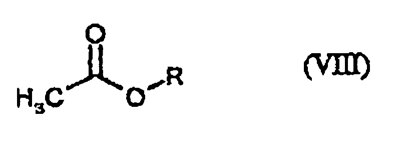 in which R is as defined above
in the presence of a base and in the presence of a diluent.

3. Process according to Claim 1 or 2, **characterized in that** compounds of the formula (II) according to Claim 1 are used in which R is C₁-C₈-alkyl.

4. Process according to Claim 1 or 2, **characterized in that** compounds of the formula (II) according to Claim 1 are used in which R is C₁-C₆-alkyl.

5. Process according to Claim 1 or 2, **characterized in that** compounds of the formula (II) according to Claim 1 are used in which R is methyl, ethyl, nor isopropyl, n-, iso-, sec- or tert-butyl and compounds of the formula (III) according to Claim 1 are used in which R¹ is methyl, ethyl, n- or isopropyl, n-, iso-, sec- or tert-butyl and compounds of the formula (V) according to Claim 1 are used in which R² and R³ independently of one another are hydrogen, methyl, ethyl, n- or isopropyl or n-, iso-, sec- or tert-butyl or together are -CH₂-CH₂-O-CH₂-CH₂-.

6. Process according to Claim 1, 2, 3, 4 or 5, **characterized in that** the first step is carried out without diluent.

7. Process according to Claim 1, 2, 3, 4, 5 or 6, **characterized in that** the hydrolysis in the third step is carried out in the presence of sulphuric acid, phosphoric acid or hydrochloric acid, each of which are optionally diluted with water.

8. Alkylphosphonic esters of the formula (IV) 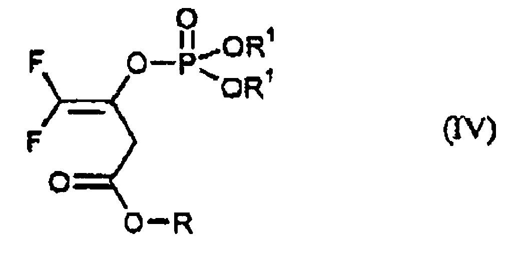 in which
R is alkyl and
R¹ is C₁-C₄-alkyl, it being possible for the radicals R¹ each to be identical or different.

9. Enamines of the formula (VI) 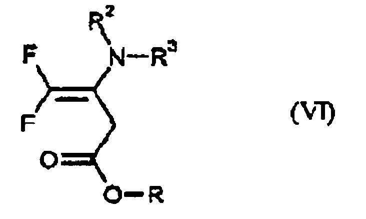 in which
R is alkyl and
R² and R³ independently of one another are hydrogen or C₁-C₈-alkyl or together are -CH₂-CH₂-O-CH₂-CH₂-, -CH₂-CH₂-S-CH₂-CH₂- or -CH₂-CH₂-N(R⁴)-CH₂-CH₂-.

## Revendications

1. Procédé de fabrication d'esters alkyliques de l'acide 4,4-difluoroacétique de formule (I) 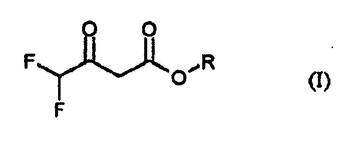 dans laquelle R représente un alkyle,
**caractérisé en ce que**
lors d'une première étape, des esters alkyliques de l'acide 4-chloro-4,4-difluoroacétique de formule (II) 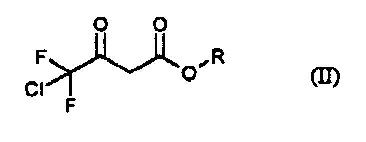 dans laquelle R a la signification donnée précédemment, sont mis en réaction avec des trialkylphosphites de formule (III)
P(OR¹)₃ (III)
dans laquelle
R¹ représente un alkyle en C₁-C₄, les radicaux R¹ pouvant chacun être identiques ou différents,
les esters de l'acide alkylphosphonique de formule (IV) ainsi obtenus 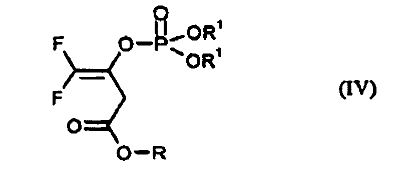 dans laquelle R et R¹ ont les significations données précédemment,
sont mis en réaction lors d'une deuxième étape avec une amine de formule (V) 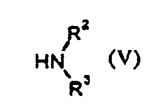 dans laquelle
R² et R³ représentent indépendamment l'un de l'autre l'hydrogène ou un alkyle en C₁-C₈ ou ensemble -CH₂-CH₂-O-CH₂-CH₂-, CH₂-CH₂-S-CH₂-CH₂- ou CH₂-CH₂-N(R⁴)-CH₂-CH₂-, éventuellement en présence d'un diluant,
et les énamines de formule (VI) ainsi obtenues 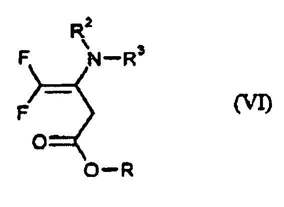 dans laquelle R, R² et R³ ont les significations données précédemment,
sont hydrolysées lors d'une troisième étape en présence d'un acide.

2. Procédé selon la revendication 1, **caractérisé en ce que** les esters alkyliques de l'acide 4-chloro-4,4-difluoroacétique de formule (II) utilisés en tant que matières premières lors de la première étape sont fabriqués par
la mise en réaction d'esters alkyliques de l'acide chlorodifluoroacétique de formule (VII) 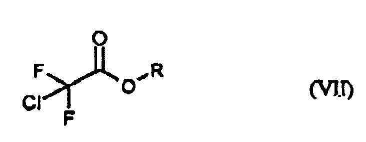 dans laquelle R a la signification donnée précédemment, avec des esters alkyliques de l'acide acétique (VIII) 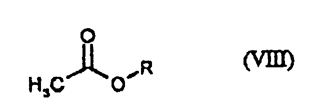 dans laquelle R a la signification donnée précédemment, en présence d'une base et en présence d'un diluant.

3. Procédé selon la revendication 1 ou 2, **caractérisé en ce que** des composés de formule (II) selon la revendication 1 dans laquelle R représente un alkyle en C₁-C₈ sont utilisés.

4. Procédé selon la revendication 1 ou 2, **caractérisé en ce que** des composés de formule (II) selon la revendication 1 dans laquelle R représente un alkyle en C₁-C₆ sont utilisés.

5. Procédé selon la revendication 1 ou 2, **caractérisé en ce que**
des composés de formule (II) selon la revendication 1 dans laquelle R représente méthyle, éthyle, n-, iso-propyle, n-, iso-, sec-, tert-butyle sont utilisés, et des composés de formule (III) selon la revendication 1 dans laquelle R¹ représente méthyle, éthyle, n-, iso-propyle, n-, iso-, sec-, tert-butyle sont utilisés, et des composés de formule (V) selon la revendication 1 dans laquelle R² et R³ représentent indépendamment l'un de l'autre hydrogène, méthyle, éthyle, n-, iso-propyle, n-, iso-, sec-, tert-butyle, ou ensemble -CH₂-CH₂-O-CH₂-CH₂- sont utilisés.

6. Procédé selon la revendication 1, 2, 3, 4 ou 5, **caractérisé en ce que** la première étape est réalisée sans diluant.

7. Procédé selon la revendication 1, 2, 3, 4, 5 ou 6, **caractérisé en ce que** l'hydrolyse lors de la troisième étape est réalisée en présence d'acide sulfurique, d'acide phosphorique ou d'acide chlorhydrique, qui sont chacun éventuellement dilués avec de l'eau.

8. Esters de l'acide alkylphosphonique de formule (IV) 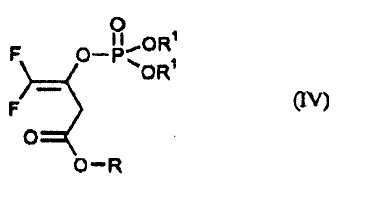 dans laquelle
R représente un alkyle,
R¹ représente un alkyle en C₁-C₄, les radicaux R¹ pouvant chacun être identiques ou différents.

9. Énamines de formule (VI) 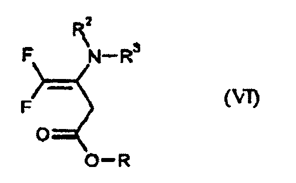 dans laquelle
R représente un alkyle,
R² et R³ représentent indépendamment l'un de l'autre l'hydrogène ou un alkyle en C₁-C₈ ou ensemble -CH₂-CH₂-O-CH₂-CH₂-, CH₂-CH₂-S-CH₂-CH₂- ou CH₂-CH₂-N(R⁴)-CH₂-CH₂-.
